(19) Europäisches Patentamt
European Patent Office
Office européen des brevets

(11) Numéro de publication : **0 077 083 B1**

(12) # FASCICULE DE BREVET EUROPÉEN

(45) Date de publication du fascicule du brevet : **07.08.85**

(51) Int. Cl.[4] : **C 07 D405/04**, C 07 D409/04, C 07 D411/04, A 61 K 31/395

(21) Numéro de dépôt : **82110740.6**

(22) Date de dépôt : **12.03.81**

(60) Numéro de publication de la demande initiale en application de l'article 76 CBE : **0046417**

(54) **Nouveaux dérivés de la thioformamide, leur préparation et les médicaments qui les contiennent.**

(30) Priorité : **18.08.80 FR 8018035**

(43) Date de publication de la demande : **20.04.83 Bulletin 83/16**

(45) Mention de la délivrance du brevet : **07.08.85 Bulletin 85/32**

(84) Etats contractants désignés : **AT BE CH DE FR GB IT LI LU NL SE**

(56) Documents cités :
**FR-A- 2 100 970**
**FR-A- 2 258 178**
**FR-A- 2 452 488**

(73) Titulaire : **RHONE-POULENC SANTE**
**Les Miroirs 18 Avenue d'Alsace**
**F-92400 Courbevoie Cedex (FR)**

(72) Inventeur : **Aloup, Jean-Claude**
**53, rue Marcel Risser**
**F-94290 Villeneuve-Le-Roi (FR)**
Inventeur : **Bouchaudon, Jean**
**23, avenue des Saules**
**F-91390 Morsang-sur-Orge (FR)**
Inventeur : **Farge, Daniel**
**30, rue des Pins Sylvestres**
**F-94320 Thiais (FR)**
Inventeur : **James, Claude**
**31 bis avenue Gambetta**
**F-75020 Paris (FR)**

(74) Mandataire : **Le Goff, Yves et al**
**RHONE-POULENC RECHERCHES Brevets Pharma**
**25, Quai Paul Doumer**
**F-92408 Courbevoie (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



Jouve, 18, rue St-Denis, 75001 Paris, France

**Description**

La présente invention concerne de nouveaux dérivés de la thioformamide de formule générale :

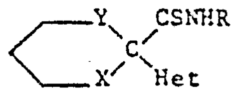

(I)

Dans la formule générale (I), R représente un atome d'hydrogène ou un radical alcoyle contenant 1 à 4 atomes de carbone en chaîne droite ou ramifiée, Het représente un radical hétérocyclique à caractère aromatique contenant un ou deux atomes d'azote tel que pyridyle-3, pyridyle-4, pyridazinyle-3, pyrazinyle ou quinolyle-3, X représente un atome de soufre ou d'oxygène et Y représente un atome de soufre ou d'oxygène, une liaison de valence ou un radical méthylène.

On connaît déjà par les demandes de brevets français publiées sous les numéros 2 100 970 et 2 258 178 des dérivés de la thioacétamide et d'acides hétérocyclylalcanethiocarboxyliques qui inhibent la sécrétion gastrique. Aucun de ces documents ne décrit ni ne suggère les produits de formule générale (I) dans laquelle X et Y ont les définitions ci-dessus.

Selon l'invention, les produits de formule générale (I) dans laquelle les symboles R, Het, X et Y sont définis comme précédemment peuvent être préparés par action d'une amine de formule générale :

$$R—NH_2 \qquad (II)$$

dans laquelle R est défini comme précédemment sur un dithioester de formule générale :

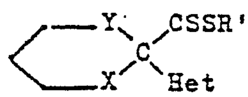

(III)

dans laquelle les symboles Het, X et Y sont définis comme précédemment et R' représente un radical alcoyle contenant 1 à 4 atomes de carbone en chaîne droite ou ramifiée ou un radical benzyle ou carboxyméthyle.

Généralement, on opère avec un excès d'amine de formule générale (II), sans solvant ou dans un solvant organique tel qu'un carbure aromatique, un éther ou un alcool à bas poids moléculaire ou un mélange de ces solvants, à une température comprise entre 20 et 130 °C, éventuellement sous pression.

Les dithioesters de formule générale (III) peuvent être obtenus par action d'un dérivé organo-lithien sur un produit de formule générale :

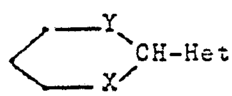

(IV)

dans laquelle Het, X et Y sont définis comme précédemment, suivie de l'action du sulfure de carbone puis d'un produit de formule générale :

$$R'—Z \qquad (V)$$

dans laquelle R' est défini comme précédemment et Z représente un atome d'halogène, de préférence un atome de chlore, de brome, ou d'iode ou un reste d'ester réactif, de préférence un reste mésyloxy ou tosyloxy.

La réaction s'effectue généralement dans un solvant organique anhydre tel que l'hexaméthylphosphorotriamide, additionné généralement d'un éther tel que le tétrahydrofuranne, à une température comprise entre − 80 et − 40 °C.

Les dérivés organo-lithiens qui conviennent particulièrement bien sont de préférence les alcoylures de lithium tels que le butyllithium et l'isopropyllithium ou le phényllithium, en solution dans un solvant inerte comme l'hexane, ou les amidures de lithium tels que le diéthylamidure de lithium ou le diisopropylamidure de lithium.

Selon les définitions de X et de Y, les produits de formule générale (IV) peuvent être préparés comme suit :

a) Les produits de formule générale (IV) dans laquelle Het est un radical $Het_1$ représentant un radical hétérocyclique à caractère aromatique contenant un ou deux atomes d'azote tel que pyridyle-3, pyridyle-4, pyridazinyle-3, pyrazinyle ou quinolyle-3, X représente un atome de soufre et Y représente une liaison de valence ou un radical méthylène, c'est-à-dire des produits de formule générale :

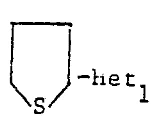

(VI) ou

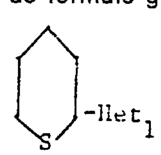

(VII)

peuvent être préparés par cyclisation au moyen d'une base organique, telle qu'un alcoolate ou un amidure alcalin, d'un dérivé de formule générale :

$$Het_1—CH_2S(CH_2)_3—Y—Z^o \qquad (IX)$$

dans laquelle les différents symboles $Het_1$, X et Y sont définis comme il vient d'être dit et $Z^o$ représente un atome d'halogène de préférence de chlore ou de brome ou un reste d'ester réactif, de préférence un reste mésyloxy ou tosyloxy, en opérant au sein d'un solvant organique anhydre, tel que le tétrahydrofuranne ou l'hexaméthylphosphorotriamide ou un mélange de ces solvants, à une température comprise entre − 80 et + 25 °C. Comme base organique, il est particulièrement avantageux d'utiliser le t.butylate de potassium, le diéthylamidure de lithium ou le diisopropylamidure de lithium.

Les dérivés hétérocycliques de formule (IX) peuvent être obtenus par hydrolyse alcaline, de préférence au moyen d'une solution aqueuse d'un hydroxyde alcalin tel que la soude, d'un sel d'une isothiourée de formule générale :

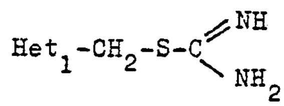

(X)

à une température comprise entre 50 °C et la température d'ébullition du mélange réactionnel, suivie de l'action d'un produit de formule générale :

$$Z'—(CH_2)_3—Y—Z' \qquad (XI)$$

dans laquelle Y est défini comme précédemment et les symboles Z', identiques ou différents, représentent chacun un atome halogène de préférence atome de chlore ou de brome, ou un reste d'ester réactif, de préférence un reste mésyloxy ou tosyloxy, à une température voisine de 20 °C en présence d'un hydroxyde alcalin tel que la soude.

Il est possible d'isoler intermédiairement le dérivé hétérocyclique de formule générale :

$$Het_1—CH_2—SH \qquad (XII)$$

provenant de l'hydrolyse alcaline de l'isothiourée de formule générale (X), puis de faire réagir le produit de formule générale (XI) en présence d'un hydroxyde alcalin tel que la soude.

Les isothiourées de formule générale (X), sous forme de sels tels que chlorhydrates, peuvent être obtenues par action de la thiourée sur un dérivé hétérocyclique de formule générale :

$$Het_1—CH_2—Z'' \qquad (XIII)$$

dans laquelle Z″ représente un atome d'halogène, de préférence un atome de chlore ou de brome, éventuellement sous forme d'un sel tel qu'un halohydrate, en opérant dans un solvant organique tel qu'un alcool (éthanol) à la température de reflux du mélange réactionnel.

Les dérivés hétérocycliques de formule (XIII) peuvent être préparés en employant, selon l'hétérocycle choisi, soit la méthode de W. MATHES et H. SCHÜLY, Angew. Chem. Intern. Ed. *2*, 144 (1963), soit la méthode de H. S. MOSHER et J. E. TESSIERI, J. Am. Chem. Soc. *73*, 4925 (1957), soit encore celles de K. Y. NOVOTSKII et coll., Khim. Getérotsikl. Soedin. (3), 412 (1970 ; C.A. *73*, 25385z (1970) ou A. HIRSCHBERG et P. E. SPOERRI, J. Org. Chem. *26*, 2356 (1961).

b) Les produits de formule générale (IV) dans laquelle Het est défini comme précédemment, X représente un atome de soufre ou d'oxygène et Y représente un atome de soufre ou d'oxygène peuvent être préparés par action d'un dérivé de formule générale :

$$HX—(CH_2)_3—Y—H \qquad (XIV)$$

sur un aldéhyde de formule générale :

$$Het—CHO \qquad (XV)$$

dans un solvant permettant d'éliminer par distillation azéotropique l'eau formée en cours de réaction. Dans la pratique, on utilise de préférence le benzène, le toluène, les xylènes ou le dichloro-1,2 éthane.

c) Les produits de formule générale (IV) dans laquelle Het est défini comme précédemment, X représente un atome d'oxygène et Y représente une liaison de valence ou un radical méthylène peuvent être préparés par cyclisation d'un produit de formule générale :

$$Het—CHOH(CH_2)_nO—R'' \qquad (XVI)$$

dans laquelle R″ est un radical protecteur de fonction alcool tel qu'un radical t. butyle, t.pentyle, ou tétrahydropyrannyle et n est égal à 3 ou 4. On opère généralement par chauffage à reflux dans un solvant tel que toluène en présence d'acide paratoluène-sulfonique ; ce traitement peut être éventuellement suivi d'un chauffage dans l'acide polyphosphorique à une température comprise entre 50 et 120 °C.

Les produits de formule générale (XVI) peuvent être préparés par action d'un dérivé magnésien de formule générale :

$$Z'''Mg—(CH_2)_nO—R'' \qquad (XVII)$$

dans laquelle R″ et n sont définis comme ci-dessus et Z‴ représente un atome d'halogène tel qu'un atome de brome ou d'iode, sur un aldéhyde de formule générale (XV), en opérant par analogie avec la méthode décrite par W. B. RENFROW, J. Org. Chem. *26*, 935 (1961).

Selon l'invention, les produits de formule générale (I) dans laquelle Het, X et Y sont définis comme précédemment et R représente un radical alcoyle contenant 1 à 4 atomes de carbone en chaîne droite ou ramifiée peuvent être obtenus par action d'un dérivé organo-lithien sur un produit de formule générale (IV) suivie de l'action d'un isothiocyanate de formule générale :

$$R'''—N{=}C{=}S \qquad (XVIII)$$

dans laquelle R‴ représente un radical alcoyle contenant 1 à 4 atomes de carbone en chaîne droite ou ramifiée.

Les dérivés organolithiens qui conviennent particulièrement bien sont de préférence les alcoylures de lithium tels que le butyllithium et l'isopropyllithium ou le phényllithium, en solution dans un solvant inerte comme l'hexane, ou les amidures tels que le diéthylamidure de lithium ou le diisopropylamidure de lithium.

Lorsque le produit de formule générale (IV) est obtenu, comme il a été dit précédemment en a), à partir d'un produit de formule générale (IX) et d'un amidure alcalin, il n'est pas nécessaire d'isoler le produit de formule générale (IV) avant de faire réagir l'isothiocyanate. Il suffit d'employer deux équivalents d'amidure.

La réaction s'effectue généralement dans un solvant organique anhydre tel que l'hexaméthylphosphorotriamide généralement additionné d'un éther tel que le tétrahydrofuranne, à une température comprise entre − 80 et − 40 °C.

Les nouveaux produits selon la présente invention peuvent être purifiés par les méthodes physiques habituelles, notamment la cristallisation et la chromatographie.

Les nouveaux produits selon l'invention présentent des propriétés pharmacologiques intéressantes associés à une faible toxicité.

Ils sont particulièrement actifs comme régulateurs du système cardiovasculaire, notamment comme antihypertenseurs. A des doses comprises entre 0,1 et 100 mg par voie orale, ils abaissent la pression artérielle chez le rat spontanément hypertendu (rat SHR)de souche CKAMOTO-AOKI. L'utilisation du rat spontanément hypertendu pour l'étude des produits anti-hypertenseurs est décrite par J. L. ROBA, Lab. Anim. Sci. *26*, 305 (1976). Leur dose létale ($DL_{50}$) chez la souris est généralement supérieure à 300 mg/kg par voie orale.

Sont plus spécialement intéressants comme anti-hypertenseurs, les produits de formule générale (I) dans laquelle le symbole Het représente le radical pyridyle-3 ou quinolyle-3.

D'un intérêt tout particulier comme anti-hypertenseurs sont :

— le N-méthyl (pyridyl-3)-2 tétrahydrothiophènecarbothioamide-2
— le N-méthyl (pyridyl-3)-2 tétrahydrothiopyrannecarbothioamide-2
— le N-méthyl (pyridyl-3)-2 oxathianne-1,3 carbiothioamide-2
— le N-méthyl (quinolyl-3)-2 tétrahydrothiophènecarbothioamide-2

Par ailleurs, les produits de formule générale (I) manifestent une activité anti-ulcère et anti-sécrétoire. Cette propriété peut être mise en évidence chez le rat, à des doses comprises entre 1 et 100 mg/kg par voie orale, en particulier dans la technique de SHAY et coll., Gastroenterology, *5*, 43 (1945).

Les exemples suivants, donnés à titre non limitatif, montrent comment l'invention peut être mise en pratique.

Dans les exemples qui suivent, les chromatographies ont été effectuées avec de la silice ayant pour granulométrie 0,063-0,20 mm ou de l'alumine ayant pour granulométrie 0,125-0,15 mm.

Exemple 1

A une solution de 14,3 g de (pyridyl-3)-2 tétrahydrothiophènecarbodithioate-2 de méthyle dans 50 $cm^3$ d'éthanol maintenue à une température voisine de 20 °C, on ajoute goutte à goute en 5 minutes 11 $cm^3$ d'une solution à 33 % (poids/volume) de méthylamine dans l'éthanol. La solution est ensuite agitée pendant 5 heures à la même température puis refroidie à 0 °C. Les cristaux apparus sont séparés par

filtration, lavés par 8 $cm^3$ d'éthanol puis 2 fois par 20 $cm^3$ au total d'oxyde d'isopropyle et séchés sous pression réduite (20 mm de mercure ; 2,7 kPa) à 20 °C. Le produit ainsi obtenu (7,2 g) est dissous dans 35 $cm^3$ d'éthanol bouillant ; la solution est additionnée de 0,4 g de noir décolorant, filtrée à chaud puis refroidie pendant 1 heure à une température voisine de 5 °C. Les cristaux apparus sont séparés par filtration, lavés par 5 $cm^3$ d'éthanol puis 2 fois par 14 $cm^3$ au total d'oxyde d'isopropyle et séchés sous pression réduite (1 mm de mercure ; 0,13 kPa) à 40 °C. On obtient ainsi 5,8 g de N-méthyl (pyridyl-3)-2 tétrahydrothiophènecarbothioamide-2 fondant à 133 °C.

Le (pyridyl-3)-2 tétrahydrothiophènecarbodithioate-2 de méthyle peut être préparé de la manière suivante :

A 170 $cm^3$ d'une solution 1,6 M de n.butyllithium dans l'hexane, maintenue sous atmosphère d'argon et refroidie à − 50 °C, on ajoute goutte à goutte en 15 minutes 125 $cm^3$ d'un mélange d'hexaméthylphosphorotriamide et de tétrahydrofuranne anhydres (47-53 en volumes). On ajoute ensuite, en 10 minutes et à − 60 °C, une solution de 30 g de (pyridyl-3)-2 tétrahydrothiophène dans 125 $cm^3$ d'un mélange d'hexaméthylphosphorotriamide et de tétrahydrofuranne anhydres (47-53 en volumes). Après 15 minutes d'agitation à − 65 °C, on ajoute en 15 minutes 20,7 g de sulfure de carbone en solution dans 16,5 $cm^3$ du mélange d'hexaméthylphosphorotriamide et de tétrahydrofuranne anhydres (47-53 en volumes). Après 5 minutes d'agitation à − 65 °C, on ajoute, en 15 minutes, à − 65 °C, 38,6 g d'iodure de méthyle en solution dans 125 $cm^3$ du mélange d'hexaméthylphosphorotriamide et de tétrahydrofuranne anhydres (47-53 en volumes). Le mélange réactionnel est ensuite agité pendant 45 minutes à cette même température puis pendant 1 heure en laissant remonter progressivement la température à 0 °C. Après addition de 1 000 $cm^3$ d'eau distillée, le mélange réactionnel est extrait 2 fois par 950 $cm^3$ au total d'acétate d'éthyle. Les extraits organiques, sont réunies et lavés 3 fois par 3 000 $cm^3$ au total d'eau distillée. Après séchage sur du sulfate de sodium anhydre, filtration et concentration à sec sous pression réduite (20 mm de mercure ; 2,7 kPa) à 60 °C, on obtient une huile brune (56,9 g) qui est chromatographiée sur 550 g de gel de silice neutre contenus dans une colonne de 5,4 cm de diamètre. On élue par 18 litres d'un mélange cyclohexane-acétate d'éthyle (95-5 en volumes) en recueillant des fractions de 1 000 $cm^3$. Les fractions 8 à 18 sont réunies et concentrées à sec sous pression réduite (20 mm de mercure ; 2,7 kPa) à 70 °C. On obtient ainsi 19,6 g de (pyridyl-3)-2 tétrahydrothiophènecarbodithioate-2 de méthyle sous la forme d'une huile orange limpide.

[Rf = 0,50 ; chromatographie sur couche mince de gel de silice ; solvant : cyclohexane-acétate d'éthyle (50-50 en volumes)].

Le (pyridyl-3)-2 tétrahydrothiophène peut être préparé de la manière suivante :

59 g de sulfure de pyridyl-3 méthyle et de chloro-3 propyle en solution dans 75 $cm^3$ de tétrahydrofuranne anhydre sont ajoutés goutte à goutte en 15 minutes et en maintenant la température en dessous de 32 °C, à une solution de 50,8 g de tertiobutylate de potassium dans un mélange de 77 $cm^3$ d'hexaméthylphosphorotriamide et de 410 $cm^3$ de tétrahydrofuranne anhydres. Après agitation pendant 1 heure à une température voisine de 20 °C, le mélange réactionnel est ajouté à un mélange de 750 $cm^3$ d'eau distillée et de 420 $cm^3$ d'éther éthylique. Après décantation, la phase aqueuse est extraite à nouveau par 200 $cm^3$ d'éther éthylique. Les phases éthérées réunies, sont lavées 3 fois par 2 100 $cm^3$ au total d'eau distillée, séchées sur du sulfate de magnésium anhydre, filtrées et concentrées à sec sous pression réduite (20 mm de mercure ; 2,7 kPa) à 45 °C. On obtient ainsi 30 g de (pyridyl-3)-2 tétrahydrothiophène sous la forme d'une huile brune.

[Rf = 0,36 ; chromatographie sur couche mince de gel de silice ; solvant : acétate d'éthyle — cyclohexane (50-50 en volumes)].

Le sulfure de pyridyl-3 méthyle et de chloro-3 propyle peut être préparé de la manière suivante :

A une solution refroidie à 12 °C de 100 g de monochlorhydrate de (pyridyl-3 méthyl)-2 isothiourée dans 250 $cm^3$ d'eau distillée on ajoute, en 10 minutes et en laissant la température remonter à 14 °C, 50 $cm^3$ d'une solution aqueuse de soude 10 N. Après chauffage pendant 30 minutes à une température de 100 °C puis refroidissement à 12 °C, on ajoute goutte à goutte et en 10 minutes 60 $cm^3$ d'une solution aqueuse de soude 10 N. On ajoute ensuite sous agitation 82 g de bromo-1 chloro-3 propane et poursuit l'agitation pendant 20 heures à une température voisine de 20 °C. Le mélange réactionnel est alors extrait 3 fois par 430 $cm^3$ au total de chlorure de méthylène. Les extraits organiques sont réunis et lavés par 250 $cm^3$ d'eau distillée puis séchés sur du sulfate de magnésium anhydre. Après filtration, la solution obtenue est versée sur 100 g de gel de silice neutre contenus dans une colonne de 3 cm de diamètre ; la colonne est ensuite éluée par 2 300 $cm^3$ de chlorure de méthylène. La première fraction (700 $cm^3$) est éliminée ; la seconde fraction (1 600 $cm^3$) est recueillie et concentrée à sec sous pression réduite (20 mm de mercure ; 2,7 kPa) à 35 °C. On obtient ainsi 59 g de sulfure de pyridyl-3 méthyle et de chloro-3 propyle sous forme d'une huile jaune.

[Rf = 0,33 ; chromatographie sur couche mince de gel de silice ; solvant : acétate d'éthyle-cyclohexane (50-50 en volumes)].

Le monochlorhydrate de (pyridyl-3 méthyl)-2 isothiourée peut être préparé de la manière suivante :

A 55 g de thiourée en suspension dans 310 $cm^3$ d'éthanol bouillant, on ajoute goutte à goutte et en 15 minutes, 100 g de chlorhydrate de chlorométhyl-3 pyridine en solution dans 310 $cm^3$ d'éthanol à 60 °C. Le mélange réactionnel est agité pendant 1 heure 45 minutes à l'ébullition puis refroidi à 30 °C. Après décantation, la solution surnageante est éliminée. Le solide gommeux restant est additionné de 400 $cm^3$

d'éthanol et agité pendant 63 heures à une température voisine de 20 °C. Les cristaux qui se sont formés sont séparés par filtration, lavés 2 fois par 60 $cm^3$ au total d'éthanol et séchés sous pression réduite (20 mm de mercure ; 2,7 kPa) à une température voisine de 20 °C. On obtient ainsi 100 g de monochlorhydrate de (pyridyl-3 méthyl)-2 isothiourée fondant à 220 °C.

Exemple 2

A une solution de 16 g de (pyridyl-4)-2 tétrahydrothiophènecarbodithioate-2 de méthyle dans 35 $cm^3$ d'éthanol maintenue à 20 °C, on ajoute goutte à goutte en 15 minutes, 12 $cm^3$ d'une solution à 33 % (poids/volume) de méthylamine dans l'éthanol. La solution est ensuite agitée pendant 1 heure à une température voisine de 20 °C puis est refroidie à 0 °C. Les cristaux apparus sont séparés par filtration, lavés 2 fois par 30 $cm^3$ au total d'éthanol et séchés sous pression réduite (20 mm de mercure ; 2,7 kPa) à une température voisine de 20 °C. On obtient ainsi 11 g de produit brut cristallisé que l'on redissout dans 100 $cm^3$ d'éthanol bouillant ; la solution obtenue est additionnée de 0,3 g de noir décolorant, filtrée à chaud puis est refroidie pendant une heure à 0 °C. Les cristaux apparus sont séparés par filtration, lavés 2 fois par 20 $cm^3$ au total d'éthanol et séchés sous pression réduite (20 mm de mercure ; 2,7 kPa) à une température voisine de 20 °C. Le produit ainsi obtenu (9 g) est chromatographié sur 20 g de gel de silice neutre contenus dans une colonne de 1,7 cm de diamètre. On élue par 1 000 $cm^3$ de chlorure de méthylène puis par 250 $cm^3$ d'un mélange chlorure de méthylène-éthanol (90-10 en volumes) en recueillant des fractions de 250 $cm^3$. Les fractions 1 à 5 sont réunies et concentrées à sec sous pression réduite (20 mm de mercure ; 2,7 kPa) à 50 °C. Le produit ainsi obtenu (8 g) est dissous dans 94 $cm^3$ d'éthanol bouillant ; la solution est additionnée de 0,4 g de noir décolorant, filtrée à chaud puis refroidie pendant 15 heures à une température voisine de 5 °C. Les cristaux apparus sont séparés par filtration, lavés 2 fois par 14 $cm^3$ au total d'éthanol et séchés sous pression réduite (20 mm de mercure ; 2,7 kPa) à une température voisine de 20 °C. Le produit ainsi obtenu (6,9 g) est dissous dans 75 $cm^3$ d'acétonitrile bouillant et la solution, additionnée de 0,2 g de noir décolorant, est filtrée à chaud puis est refroidie pendant 1 heure à 0 °C. Les cristaux apparus sont séparés par filtration, lavés par 7 $cm^3$ d'acétonitrile et séchés sous pression réduite (1 mm de mercure ; 0,13 kPa) à 60 °C. On obtient ainsi 5,9 g de N-méthyl (pyridyl-4)-2 tétrahydrothiophènecarbothioamide-2 fondant à 178 °C.

Le (pyridyl-4)-2 tétrahydrothiophènecarbotithioate-2 de méthyle peut être préparé de la manière suivante :

A 265 $cm^3$ d'une solution 1,6 M de n-butyllithium dans l'hexane maintenue sous atmosphère d'azote à une température voisine de − 60 °C, on ajoute goutte à goutte et en 15 minutes, 192 $cm^3$ d'un mélange d'hexaméthylphosphorotriamide et de tétrahydrofuranne anhydres (47-53 en volumes). On ajoute ensuite en 20 minutes une solution de 46,7 g de (pyridyl-4)-2 tétrahydrothiophène dans 192 $cm^3$ du mélange d'hexaméthylphosphorotriamide et de tétrahydrofuranne anhydres (47-53 en volumes). Après 20 minutes d'agitation à la même température, on ajoute en 20 minutes une solution de 32 g de sulfure de carbone dans 192 $cm^3$ du mélange d'hexaméthylphosphorotriamide et de tétrahydrofuranne anhydres (47-53 en volumes). Après 5 minutes d'agitation à la même température, on ajoute en 15 minutes une solution de 60,5 g d'iodure de méthyle dans 192 $cm^3$ du mélange d'hexaméthylphosphorotriamide et de tétrahydrofuranne anhydres (47-53 en volumes). Le mélange réactionnel est ensuite agité pendant 45 minutes à − 60 °C puis pendant 25 minutes en laissant remonter progressivement la température à 5 °C. Il est ensuite coulé sur un mélange de 1 500 $cm^3$ d'eau distillée et de 900 $cm^3$ d'acétate d'éthyle.

Après décantation, la solution aqueuse est extraite par 500 $cm^3$ d'acétate d'éthyle. Les phases organiques sont réunies et lavées 3 fois par 4 500 $cm^3$ au total d'eau distillée puis 2 fois par 250 $cm^3$ au total d'une solution aqueuse 2 N d'acide chlorhydrique. Les extraits organiques sont éliminés ; les extraits aqueux sont lavés 2 fois par 300 $cm^3$ au total d'acétate d'éthyle, puis neutralisés à pH = 8 par addition de bicarbonate de sodium ; ils sont extraits 3 fois par 600 $cm^3$ au total d'acétate d'éthyle. Ces nouveaux extraits organiques sont réunis et lavés 3 fois par 600 $cm^3$ au total d'eau distillée, séchés sur du sulfate de sodium anhydre, filtrés et concentrés à sec sous pression réduite (20 mm de mercure ; 2,7 kPa) à 50 °C. Le produit obtenu (58 g) est chromatographié sur 450 g de gel de silice neutre contenu dans une colonne de 5 cm de diamètre ; la colonne est ensuite éluée par 1 500 $cm^3$ d'un mélange cyclohexane-acétate d'éthyle (95-5 en volumes), par 4 000 $cm^3$ d'un mélange cyclohexane-acétate d'éthyle (90-10 en volumes), par 2 000 $cm^3$ d'un mélange cyclohexane-acétate d'éthyle (85-15 en volumes), et par 2 500 $cm^3$ d'un mélange cyclohexane-acétate d'éthyle (80-20 en volumes) en recueillant des fractions de 500 $cm^3$. Les fractions 10 à 12 sont réunies et concentrées à sec (20 mm de mercure ; 2,7 kPa) à 50 °C et le produit partiellement cristallisé obtenu (10 g) est filtré, lavé 2 fois par 30 $cm^3$ au total d'oxyde d'isopropyle et séché sous pression réduite (20 mm de mercure ; 2,7 kPa) à une température voisine de 20 °C ; on obtient ainsi un premier lot de 6 g de produit. Les fractions 13 à 20 sont réunies et concentrées à sec sous pression réduite (20 mm de mercure ; 2,7 kPa) à 50 °C. Le second lot obtenu (34 g) est réuni avec le premier lot de 6 g et dissous dans 180 $cm^3$ d'oxyde d'isopropyle bouillant ; la solution est additionnée de 0,4 g de noir décolorant, filtrée à chaud puis refroidie pendant 1 heure à 0 °C. Les cristaux apparus sont séparés par filtration, lavés 2 fois par 52 $cm^3$ au total d'oxyde d'isopropyle et séchés sous pression réduite (1 mm de mercure ; 0,13 kPa) à 35 °C. On obtient ainsi 28,3 g de (pyridyl-4)-2 tétrahydrotiophène-carbodithioate-2 de méthyle fondant à 64 °C.

Le (pyridyl-4)-2 tétrahydrothiophène peut être préparé de la manière suivante :

Une solution de 81 g de sulfure de pyridyl-4 méthyle et de chloro-3 propyle dans 100 $cm^3$ de tétrahydrofuranne anhydre est ajoutée goutte à goutte, en 20 minutes, et en maintenant la température en dessous de 33 °C, à une solution de 69,5 g de tertiobutylate de potassium dans un mélange de 108 $cm^3$ d'hexaméthylphosphorotriamide et de 560 $cm^3$ de tétrahydrofuranne anhydres. Le mélange réactionnel est ensuite agité pendant 1 heure 30 minutes à une température voisine de 20 °C ; il est ensuite coulé sur un mélange de 1 000 $cm^3$ d'eau distillée et de 600 $cm^3$ d'éther éthylique. Après décantation, la phase aqueuse est extraite 2 fois par 400 $cm^3$ au total d'éther éthylique. Les extraits éthérés sont réunies, lavés 3 fois par 3 000 $cm^3$ au total d'eau distillée, séchés sur du sulfate de sodium anhydre, filtrés et concentrés à sec sous pression réduite (20 mm de mercure ; 2,7 kPa) à 40 °C. On obtient ainsi 46,7 g de (pyridyl-4)-2 tétrahydrothiophène sous forme d'une huile brune.

[Rf = 0,35 ; chromatographie sur couche mince de gel de silice ; solvant : cyclohexane-acétate d'éthyle (50-50 en volumes)].

Le sulfure de pyridyl-4 méthyle et de chloro-3 propyle peut être préparé de la manière suivante :

A une solution de 116,5 g de dichlorhydrate de (pyridyl-4 méthyl)-2 isothiourée dans 240 $cm^3$ d'eau distillée, on ajoute goutte à goutte, en 15 minutes et en maintenant la température en dessous de 14 °C, 97 $cm^3$ d'une solution aqueuse de soude 10 N. Après chauffage pendant 20 minutes à 83 °C puis refroidissement à 12 °C, on ajoute sous agitation 60 $cm^3$ de solution aqueuse de soude 10 N puis 81,5 g de bromo-1 chloro-3 propane et poursuit l'agitation pendant 15 heures à une température voisine de 20 °C. Le mélange réactionnel est alors extrait 3 fois par 430 $cm^3$ au total de chlorure de méthylène ; les extraits organiques sont réunis, lavés par 250 $cm^3$ d'eau distillée et séchés sur du sulfate de sodium anhydre. Après filtration, la solution obtenue est versée sur 125 g de gel de silice neutre contenus dans une colonne de 3,2 cm de diamètre ; la colonne est ensuite éluée par 1 600 $cm^3$ de chlorure de méthylène. La première fraction (400 $cm^3$) est éliminée ; la seconde fraction (1 200 $cm^3$) est recueillie et concentrée à sec sous pression réduite (20 mm de mercure ; 2,7 kPa) à 40 °C. On obtient ainsi 81 g de sulfure de pyridyl-4 méthyle et de chloro-3 propyle sous la forme d'une huile brune.

[Rf = 0,3 ; chromatographie sur couche mince de gel de silice ; solvant : cyclohexane-acétate d'éthyle (50-50 en volumes)].

Le dichlorhydrate de (pyridyl-4 méthyl)-2 isothiourée peut être préparé de la manière suivante :

A une suspension de 45,5 g de thiourée dans 260 $cm^3$ d'éthanol bouillant, on ajoute goutte à goutte et en 30 minutes, 82 g de chlorhydrate de chlorométhyl-4 pyridine en suspension dans 260 $cm^3$ d'éthanol à faible ébullition. Le mélange réactionnel est agité pendant 1 heure 30 minutes à l'ébullition puis refroidi à 5 °C. Les cristaux apparus sont séparés par filtration, lavés 2 fois par 60 $cm^3$ au total d'éthanol et séchés sous pression réduite (20 mm de mercure ; 2,7 kPa) à une température voisine de 20 °C. On obtient ainsi 116 g de dichlorhydrate de (pyridyl-4 méthyl)-2 isothiourée fondant à 260 °C.

## Exemple 3

A 119 $cm^3$ d'une solution 1,6 M de n.butyllithium dans l'hexane maintenue sous atmosphère d'azote et à une température voisine de − 60 °C, on ajoute goutte à goutte et en 15 minutes, 70 $cm^3$ d'un mélange d'hexaméthylphosphorotriamide et de tétrahydrofuranne anhydres (47-53 en volumes). On ajoute ensuite en 15 minutes une solution de 19,5 g de pyrazinyl-2 tétrahydrothiophène dans 70 $cm^3$ du mélange d'hexaméthylphosphorotriamide et de tétrahydrofuranne anhydres (47-53 en volumes). Après 15 minutes d'agitation, on ajoute en 15 minutes une solution de 12,7 g d'isothiocyanate de méthyle dans 30 $cm^3$ du mélange d'hexaméthylphosphorotriamide et de tétrahydrofuranne anhydres (47-53 en volumes). Le mélange réactionnel est ensuite agité pendant 45 minutes à − 65 °C puis pendant 1 heure en laissant remonter progressivement la température à 20 °C environ. Il est ensuite coulé sur un mélange de 300 $cm^3$ d'acétate d'éthyle et de 500 $cm^3$ d'eau distillée. Après décantation, la solution aqueuse est extraite 2 fois par 400 $cm^3$ au total d'acétate d'éthyle. Les extraits organiques sont réunis, lavés 3 fois par 1 500 $cm^3$ au total d'eau distillée, séchés sur du sulfate de sodium anhydre, filtrés et concentrés sous pression réduite (20 mm de mercure ; 2,7 kPa) à 70 °C. Le produit obtenu (33,3 g) est chromatographié sur 33 g de gel de silice neutre contenus dans une colonne de 4,2 cm de diamètre. On élue successivement avec 4 000 $cm^3$ d'un mélange cyclohexane-acétate d'éthyle (90-10 en volumes) puis avec 7 000 $cm^3$ d'un mélange cyclohexane-acétate d'éthyle (80-20 en volumes) en recueillant des fractions de 500 $cm^3$. Les fractions 13 à 22 sont réunies et concentrées à sec sous pression réduite (20 mm de mercure ; 2,7 kPa) à 60 °C. Le produit obtenu (11 g) est dissous dans 48 $cm^3$ d'un mélange bouillant de propanol et d'oxyde d'isopropyle (75-25 en volumes) ; la solution est additionnée de 0,2 g de noir décolorant, filtrée à chaud puis refroidie pendant 18 heures à 5 °C. Les cristaux apparus sont séparés par filtration, lavés par 10 $cm^3$ du mélange propanoloxyde d'isopropyle (75-25 en volumes) et séchés sous pression réduite (1 mm de mercure ; 0,13 kPa) à 45 °C. On obtient ainsi 4,9 g de N-méthyl pyrazinyl-2 tétrahydrothiophènecarbothioamide-2 fondant à 127 °C.

Le pyrazinyl-2 tétrahydrothiophène peut être préparé de la manière suivante :

Une solution de 59 g de sulfure de pyrazinylméthyle et de chloro-3 propyle dans 75 $cm^3$ de tétrahydrofuranne anhydre est ajoutée goutte à goutte, en 15 minutes et en maintenant la température en dessous de 30 °C, à une solution de 51 g de tertiobutylate de potassium dans un mélange de 75 $cm^3$

d'hexaméthylphosphorotriamide et de 400 $cm^3$ de tétrahydrofuranne anhydres. Après 30 minutes d'agitation à la même température, on ajoute 10 g de tertiobutylate de potassium et poursuit l'agitation pendant 30 minutes. Le mélange réactionnel est ensuite coulé sur un mélange de 800 $cm^3$ d'eau distillée et de 400 $cm^3$ d'éther éthylique. Après décantation, la phase aqueuse est extraite 2 fois par 300 $cm^3$ au total d'éther éthylique. Les extraits organiques sont réunis, lavés 3 fois par 2 400 $cm^3$ au total d'eau distillée, séchés sur du sulfate de sodium anhydre, filtrés et concentrés à sec sous pression réduite (20 mm de mercure ; 2,7 kPa) à 35 °C. Le produit obtenu (30 g) est chromatographié sur 100 g de gel de silice neutre contenus dans une colonne de 3 cm de diamètre ; la colonne est éluée par 750 $cm^3$ de cyclohexane, par 2 500 $cm^3$ d'un mélange cyclohexane-acétate d'éthyle (90-10 en volumes) et par 500 $cm^3$ d'acétate d'éthyle en recueillant des fractions de 250 $cm^3$. Les fractions 4 à 15 sont réunies et concentrées à sec sous pression réduite (20 mm de mercure ; 2,7 kPa) à 35 °C. On obtient ainsi 19,5 g de pyrazinyl-2 tétrahydrothiophène sous la forme d'une huile brune.

[Rf = 0,52 ; chromatographie sur couche mince de gel de silice ; solvant : cyclohexane-acétate d'éthyle (50-50 en volumes)].

Le sulfure de pyrazinylméthyle et de chloro-3 propyle peut être préparé de la manière suivante :

A une solution de 77,6 g de monochlorhydrate de (pyrazinylméthyl)-2 isothiourée dans 250 $cm^3$ d'eau distillée, on ajoute goutte à goutte, en 15 minutes et à une température voisine de 10 °C, 39 $cm^3$ d'une solution aqueuse de soude 10N. Après chauffage pendant 30 minutes à 70 °C puis refroidissement à 15 °C, on ajoute 47 $cm^3$ de solution aqueuse de soude 10N puis 66,5 g de bromo-1 chloro-3 propane et l'on poursuit l'agitation pendant 15 heures à une température voisine de 20 °C. Le mélange réactionnel est ensuite extrait 3 fois par 280 $cm^3$ au total de chlorure de méthylène. Les extraits organiques sont réunis, lavés 2 fois par 200 $cm^3$ au total d'eau distillée, séchés sur du sulfate de sodium anhydre et filtrés. La solution obtenue est versée sur 80 g de gel de silice neutre contenus dans une colonne de 2,7 cm de diamètre ; la colonne est ensuite éluée par 900 $cm^3$ de chlorure de méthylène. La première fraction (300 $mc^3$) est éliminée ; la seconde fraction (600 $cm^3$) est recueillie et concentrée à sec sous pression réduite (20 mm de mercure ; 2,7 kPa) à 35 °C. On obtient ainsi 59 g de sulfure de pyrazinylméthyle et de chloro-3 propyle sous forme d'une huile jaune-orangé.

[Rf = 0,47 ; chromatographie sur couche mince de gel de silice ; solvant ; cyclohexane-acétate d'éthyle (50-50 en volumes)].

Le chlorhydrate de (pyrazinylméthyl)-2 isothiourée peut être préparé de la manière suivante :

A une suspension de 68 g de thiourée dans 370 $cm^3$ d'éthanol bouillant, on ajoute goutte à goutte et en 15 minutes une solution de 88 g de chlorométhylpyrazine dans 200 $cm^3$ d'éthanol. Après 1 heure 30 minutes d'agitation à l'ébullition, le mélange réactionnel est refroidi pendant 15 heures à une température voisine de 5 °C. Les cristaux apparus sont séparés par filtration, lavés 2 fois par 120 $cm^3$ d'éthanol et séchés sous pression réduite (20 mm de mercure ; 2,7 kPa) à une température voisine de 20 °C. On obtient ainsi 74 g de chlorhydrate de (pyrazinylméthyl)-2 isothiourée fondant à 183 °C.

La chlorométhylpyrazine peut être préparée comme décrit dans la littérature [A. HIRSCHBERT et P. Spoerri, J. Org. Chem., *26*, 2356 (1961)].

## Exemple 4

A 55 $cm^3$ d'une solution 1,6 M de n.butyllithium dans l'hexane, maintenue sous atmosphère d'azote à une température voisine de − 60 °C, on ajoute goutte à goutte et en 15 minutes, 36 $cm^3$ d'un mélange d'hexaméthylphosphorotriamide et de tétrahydrofuranne anhydres (47-53 en volumes). On ajoute ensuite en 20 minutes une solution de 9,7 g de (pyridazinyl-3)-2 tétrahydrothiophène dans 36 $cm^3$ du mélange d'hexaméthylphosphorotriamide et de tétrahydrofuranne anhydres (47-53 en volumes). Après 15 minutes d'agitation à la même température, on ajoute en 15 minutes 6,4 g d'isothiocyanate de méthyle en solution dans 36 $cm^3$ du mélange d'hexaméthylphosphorotriamide et de tétrahydrofuranne anhydres (47-53 en volumes). Le mélange réactionnel est ensuite agité pendant 1 heure à − 65 °C puis pendant une heure en laissant remonter progressivement la température à − 10 °C. Il est ensuite coulé sur un mélange de 350 $cm^3$ d'eau distillée et de 300 $cm^3$ d'acétate d'éthyle. Après décantation, la solution aqueuse est extraite par 200 $cm^3$ d'acétate d'éthyle. Les extraits organiques sont réunis, lavés 3 fois par 900 $cm^3$ au total d'eau distillée, séchés sur du sulfate de sodium anhydre, filtrés et concentrés à sec sous pression réduite (20 mm de mercure ; 2,7 kPa) à 55 °C. Le produit obtenu (12,4 g) est réuni à 1 g de produit préparé dans les mêmes conditions, et chromatographié sur 154 g de gel de silice neutre contenus dans une colonne de 3,2 cm de diamètre. La colonne est éluée par 500 $cm^3$ d'un mélange cyclohexane-acétate d'éthyle (90-10 en volumes), 1 000 $cm^3$ d'un mélange cyclohexane-acétate d'éthyle (80-20 en volumes), 2 250 $cm^3$ d'un mélange cyclohexane-acétate d'éthyle (70-30 en volumes) et 2 500 $cm^3$ d'un mélange cyclohexane-acétate d'éthyle (50-50 en volumes) en recueillant des fractions de 250 $cm^3$. Les fractions 19 à 25 sont réunies et concentrées à sec sous pression réduite (20 mm de mercure ; 2,7 kPa) à 55 °C. Le produit obtenu (2,4 g) est dissous dans 50 $cm^3$ d'éthanol bouillant ; la solution est additionnée de 0,15 g de noir décolorant et filtrée à chaud puis est refroidie pendant 30 minutes à 0 °C. Les cristaux apparus sont séparés par filtration, lavés par 2 $cm^3$ d'éthanol puis 2 fois par 6 $cm^3$ au total d'oxyde d'isopropyle et séchés sous pression réduite (1 mm de mercure ; 0,13 kPa) à 60 °C. On obtient ainsi 0,8 g de N-méthyl (pyridazinyl-3)-2 tétrahydrothiophènecarbothioamide-2 fondant à 199 °C.

Le (pyridazinyl-3)-2 tétrahydrothiophène peut être préparé de la manière suivante :

Une solution de 28,2 g de sulfure de pyridazinyl-3 méthyle et de chloro-3 propyle dans 35 $cm^3$ de tétrahydrofuranne anhydre est ajoutée goutte à goutte, en 15 minutes et en maintenant la température en dessous de − 20 °C, à une solution de 24 g de tertiobutylate de potassium dans un mélange de 35 $cm^3$ d'hexaméthylphosphorotriamide et de 190 $cm^3$ de tétrahydrofuranne anhydres. Le mélange réactionnel est ensuite agité pendant 1 heure 30 minutes à − 40 °C puis il est coulé à 0 °C sur un mélange de 500 $cm^3$ d'eau distillée et de 500 $cm^3$ d'éther éthylique. Après décantation, la phase aqueuse est extraite par 250 $cm^3$ d'éther éthylique. Les extraits éthérés sont réunis, lavés 3 fois par 1 500 $cm^3$ au total d'eau distillée, séchés sur du sulfate de sodium anhydre, filtrés et concentrés à sec sous pression réduite (20 mm de mercure ; 2,7 kPa) à 30 °C. On obtient ainsi 10,5 g de (pyridazinyl-3)-2 tétrahydrothiophène sous forme d'une huile brune.

[Rf = 0,4 ; chromatographie sur couche mince de gel de silice ; solvant : acétate d'éthyle].

Le sulfure de pyridazinyl-3 méthyle et de chloro-3 propyle peut être préparé de la manière suivante :

A une solution de 106 g de dichlorhydrate de (pyridazinyl-3 méthyl)-2 isothiourée dans 220 $cm^3$ d'eau distillée, on ajoute goutte à goutte, en 10 minutes et en maintenant la température en dessous de 18 °C, 84 $cm^3$ d'une solution aqueuse de soude 10 N. Après chauffage pendant 20 minutes à 75 °C puis refroidissement à 10 °C, on ajoute sous agitation 50 $cm^3$ de solution aqueuse de soude 10 N puis 69 g de bromo-1 chloro-3 propane et l'on poursuit l'agitation pendant 15 heures à une température voisine de 20 °C. Le mélange réactionnel est alors extrait 3 fois par 340 $cm^3$ au total de chlorure de méthylène. Les extraits organiques sont réunis, lavés par 250 $cm^3$ d'eau distillée et séchés sur du sulfate de sodium anhydre. Après filtration, la solution obtenue est versée sur 80 g de gel de silice neutre contenus dans une colonne de 2,7 cm de diamètre ; la colonne est ensuite éluée par 700 $cm^3$ de chlorure de méthylène. La première fraction (200 $cm^3$) est éliminée. La seconde fraction (500 $cm^3$) est recueillie et concentrée à sec sous pression réduite (20 mm de mercure, 2,7 kPa) à 35 °C. On obtient ainsi 60 g de sulfure de pyridazinyl-3 méthyle et de chloro-3 propyle sous forme d'une huile rouge.

[Rf = 0,1 ; chromatographie sur couche mince de gel de silice ; solvant : cyclohexane-acétate d'éthyle (50-50 en volumes)].

Le dichlorhydrate de (pyridazinyl-3 méthyl)-2 isothiourée peut être préparé comme décrit dans la littérature [NOVITSKII, K. YU. et coll., Khim. Geterotsikl. Soedin., (3) 412 (1970) ; CA *73*, 25385z (1970)].

## Exemple 5

A 44 $cm^3$ d'une solution 1,6 M de n-butyllithium dans l'hexane, maintenue sous atmosphère d'azote et refroidie à − 70 °C, on ajoute, goutte à goutte et en 10 minutes, 50 $cm^3$ d'un mélange d'hexaméthylphosphorotriamide et de tétrahydrofuranne anhydres (47-53 en volumes). On ajoute ensuite en 25 minutes à la même température une solution de 8,5 g de (pyridyl)-3)-2 oxathianne-1,3 dans 50 $cm^3$ du mélange d'hexaméthylphosphorotriamide et de tétrahydrofuranne anhydres (47-53 en volumes). Après 15 minutes d'agitation, on ajoute en 15 minutes une solution de 5,4 g d'isothiocyanate de méthyle dans 50 $cm^3$ du mélange d'hexaméthylphosphorotriamide et de tétrahydrofuranne anhydres (47-53 en volumes). Le mélange réactionnel est agité pendant 45 minutes à − 70 °C puis pendant 1 heure en laissant remonter progressivement la température à 20 °C environ. Il est ensuite coulé sur un mélange agité de 300 $cm^3$ d'eau distillée et de 240 $cm^3$ d'acétate d'éthyle. Après décantation, la phase aqueuse est extraite 2 fois par 400 $cm^3$ au total d'acétate d'éthyle. Les phases organiques sont réunies, lavées 3 fois par 600 $cm^3$ au total d'eau distillée, séchées sur du sulfate de sodium anhydre, filtrées et concentrées à sec sous pression réduite (20 mm de mercure ; 2,7 kPa) à 50 °C. Le produit obtenu, additionné de 70 $cm^3$ d'un mélange d'oxyde d'isopropyle et d'acétate d'éthyle (90-10 en volumes), est agité pendant 10 minutes à 20 °C. Les cristaux apparus sont séparés par filtration, lavés 2 fois par 20 $cm^3$ au total d'oxyde d'isopropyle, et séchés sous pression réduite (20 mm de mercure ; 2,7 kPa) à une température voisine de 20 °C. Le produit obtenu (4,4 g) est chromatographié sur 40 g de gel de silice neutre contenu dans une colonne de 2,6 cm de diamètre. On élue avec 2 000 $cm^3$ d'un mélange cyclohexane-acétate d'éthyle (40-60 en volumes) puis avec 800 $cm^3$ d'acétate d'éthyle, en recueillant des fractions de 200 $cm^3$. Les fractions 4 à 14 sont réunies et concentrées à sec sous pression réduite (20 mm de mercure ; 2,7 kPa) à 50 °C. Le produit obtenu (3,9 g) est dissous dans 45 $cm^3$ d'acétonitrile bouillant. La solution, additionnée de 0,2 g de noir décolorant, est filtrée à chaud puis conservée après refroidissement, pendant 1 heure à 0 °C. Les cristaux apparus sont séparés par filtration, lavés 2 fois par 10 $cm^3$ au total d'acétonitrile et séchés sous pression réduite (1 mm de mercure ; 0,13 kPa) à 60 °C. On obtient ainsi 2,9 g de N-méthyl (pyridyl-3)-2 oxathianne-1,3 carbothioamide-2 fondant à 194 °C.

Le (pyridyl-3)-2 oxathianne-1,3 peut être préparé de la façon suivante :

Une solution de 16 g de pyridinecarboxaldéhyde-3, de 48 g de mercapto-3 propanol-1 et de 2,25 g d'acide paratoluènesulfonique dans 1 500 $cm^3$ de dichloro-1,2 éthane est maintenue à l'ébullition pendant 15 heures de façon à éliminer par azéotropie l'eau formée. Après refroidissement à une température voisine de 20 °C, le mélange réactionnel est lavé 3 fois par 750 $cm^3$ au total d'une solution aqueuse 5 N de soude puis 3 fois par 1 500 $cm^3$ au total d'eau distillée. La phase organique est séchée sur du sulfate de sodium anhydre, filtrée et concentrée à sec sous pression réduite (20 mm de mercure ; 2,7 kPa) à 50 °C. Le produit obtenu (15,6 g) est chromatographié sur 200 g de gel de silice neutre contenus

dans une colonne de 3,7 cm de diamètre. On élue successivement avec 600 $cm^3$ d'un mélange cyclohexane-acétate d'éthyle (80-20 en volumes), 2 700 $cm^3$ d'un mélange cyclohexane-acétate d'éthyle (70-30 en volumes), en recueillant des fractions de 300 $cm^3$. Les fractions 5 à 11 sont réunies et concentrées à sec sous pression réduite (20 mm de mercure ; 2,7 kPa) à 50 °C. On obtient ainsi 8,6 g de (pyridyl-3)-2 oxathianne-1,3 sous la forme d'une huile jaune.

[Rf = 0,6 ; chromatographie sur couche mince de gel de silice ; solvant : acétate d'éthyle].

Le mercapto-3 propanol-1 peut être préparé comme décrit dans la littérature [R. O. CLINTON et Coll. J. Am. Chem. Soc., *67*, 594 (1945)].

## Exemple 6

A 625 $cm^3$ d'une solution 1,6 M de n-butyllithium dans l'hexane, maintenue sous atmosphère d'azote et refroidie à − 60 °C, on ajoute goutte à goutte en 10 minutes une solution de 101 g de diisopropylamine dans 225 $cm^3$ d'un mélange d'hexaméthylphoisphorotriamide et de tétrahydrofuranne anhydres (47-53 en volumes). Après 10 minutes d'agitation, on ajoute en 30 minutes à la même température une solution de 84 g de sulfure de pyridyl-3 méthyle et de chloro-4 butyle dans 300 $cm^3$ du mélange d'hexaméthylphosphorotriamide et de tétrahydrofuranne anhydre (47-53 en volumes). Le mélange réactionnel est agité pendant 1 heure à une température voisine de − 65 °C puis on coule en 20 minutes à cette température une solution de 117 g d'isothiocyanate de méthyle dans 225 $cm^3$ du mélange d'hexaméthylphosphorotriamide et de tétrahydrofuranne anhydres (47-53 en volumes). Le mélange est ensuite agité pendant 30 minutes à − 60 °C puis pendant 1 heure en laissant remonter progressivement la température à 10 °C. Après addition de 1 litre d'eau distillée, le mélange est extrait 6 fois par 5 litres au total d'acétate d'éthyle. Les extraits organiques sont réunis, lavés 5 fois par 5 litres au total d'eau distillée, séchés sur du sulfate de sodium anhydre, filtrés et concentrés à sec sous pression réduite (30 mm de mercure ; 4 kPa) à 40 °C. Le produit obtenu (167 g) est chromatographié sur 1 500 g de gel de silice neutre contenus dans une colonne de 7,4 cm de diamètre.

On élimine les impuretés en éluant avec environ 50 litres d'un mélange de cyclohexane et d'acétate d'éthyle dont la teneur en acétate d'éthyle varie progressivement de 0 à 30 %. On élue ensuite par de l'acétate d'éthyle pur en recueillant 3 fractions de 1 litre puis 8 fractions de 0,7 litre. Ces 8 dernières fractions sont réunies et concentrées à sec sous pression réduite (20 mm de mercure ; 2,7 kPa) à 60 °C. Le produit obtenu (20 g) est dissous dans un mélange bouillant de 80 $cm^3$ d'oxyde d'isopropyle et de 40 $cm^3$ d'éthanol. Après refroidissement pendant 1 heure à 0 °C, les cristaux apparus sont séparés par filtration, lavés par 25 $cm^3$ d'oxyde d'isopropyle et séchés sous pression réduite (20 mm de mercure ; 2,7 kPa) à une température voisine de 20 °C. Le produit obtenu (14,2 g) est dissous dans 43 $cm^3$ d'acétonitrile bouillant ; la solution, additionnée de 0,8 g de noir décolorant, est filtrée à chaud et conservée, après refroidissement, pendant 45 minutes à une température voisine de 5 °C. Les cristaux apparus sont séparés par filtration, lavés par 4 $cm^3$ d'acétonitrile puis 2 fois par 8 $cm^3$ au total d'oxyde d'isopropyle. Après séchage sous pression réduite (1 mm de mercure ; 0,13 kPa) à 60 °C, on obtient 10,6 g de N-méthyl (pyridyl-3)-2 tétrahydrothiopyrannecarbothioamide-2 fondant à 131 °C.

Le sulfure de pyridyl-3 méthyle et de chloro-4 butyle peut être préparé de la manière suivante :

A une solution de 173 g de dichlorhydrate de (pyridyl-3 méthyl)-2 isothiourée dans 330 $cm^3$ d'eau distillée refroidie à 4 °C, on ajoute, goutte à goutte en 25 minutes et en maintenant la température en dessous de 12 °C, 144 $cm^3$ d'une solution aqueuse de soude 10 N. Après chauffage pendant 25 minutes à 70 °C puis refroidissement à 11 °C, on ajoute sous agitation 89 $cm^3$ de solution aqueuse de soude 10 N puis 123,5 g de bromo-1 chloro-4 butane et poursuit l'agitation pendant 15 heures à une température voisine de 20 °C. Le mélange réactionnel est alors extrait 4 fois par 560 $cm^3$ au total de chlorure de méthylène ; les extraits organiques sont réunis, lavés 2 fois par 800 $cm^3$ au total d'eau distillée, séchés sur du sulfate de sodium anhydre et filtrés. La solution est versée sur 150 g de gel de silice neutre contenus dans une colonne de 3,7 cm de diamètre ; la colonne est éluée par 2 500 $cm^3$ de chlorure de méthylène. La première fraction (600 $cm^3$) est éliminée. La seconde (2 500 $cm^3$) est concentrée à sec sous pression réduite (25 mm de mercure ; 3,4 kPa) sans dépasser 30 °C. On obtient ainsi 110 g de sulfure de pyridyl-3 méthyle et de chloro-4 butyle.

[Rf = 0,31 ; chromatographie sur couche mince de gel de silice ; solvant : cyclohexane-acétate d'éthyle (50-50 en volumes)].

Le dichlorhydrate de (pyridyl-3 méthyl)-2 isothiourée peut être préparé de la manière suivante :

A une solution de 91 g de thiourée dans 510 $cm^3$ d'éthanol bouillant, on ajoute, goutte à goutte et en 20 minutes, 164 g de chlorhydrate de chlorométhyl-3 pyridine en solution dans 510 $cm^3$ d'éthanol à 50 °C. Le mélange réactionnel est agité pendant 3 heures 50 minutes à l'ébullition puis refroidi à 26 °C. Les cristaux apparus sont séparés par filtration, lavés 2 fois par 400 $cm^3$ au total d'éthanol et séchés sous pression réduite (25 mm de mercure ; 2,7 kPa) à une température voisine de 20 °C. On obtient ainsi 173 g de dichlorhydrate de (pyridyl-3 méthyl)-2 isothiourée fondant à 212 °C.

## Exemple 7

A 59 $cm^3$ d'une solution 1,6 M de n-butyllithium dans l'hexane, maintenue sous atmosphère d'azote

et refroidie à – 50 °C, on ajoute, goutte à goutte en 11 minutes, une solution de 9,6 g de diisopropylamine dans 22 $cm^3$ d'un mélange d'hexaméthylphosphorotriamide et de tétrahydrofuranne anhydres (47-53 en volumes). On ajoute ensuite en 20 minutes à une température voisine de – 60 °C une solution de 9,2 g de sulfure de quinolyl-3 méthyle et de chloro-3 propyle dans 28 $cm^3$ du mélange d'hexaméthylphosphorotriamide et de tétrahydrofuranne anhydres (47-53 en volumes). Le mélange réactionnel est agité pendant 30 minutes à la même température puis on coule en 15 minutes une solution de 11,1 g d'isothiocyanate de méthyle dans 22 $cm^3$ du mélange d'hexaméthylphosphorotriamide et de tétrahydrofuranne anhydres (47-53 en volumes). Le mélange est encore agité pendant 25 minutes à – 65 °C puis pendant 30 minutes en laissant remonter progressivement la température à 10 °C. Après addition de 100 $cm^3$ d'eau distillée, le mélange est extrait 3 fois par 300 $cm^3$ au total d'acétate d'éthyle. Les extraits organiques sont réunis, lavés 5 fois par 500 $cm^3$ au total d'eau distillée, séchés sur du sulfate de sodium anhydre et concentrés à sec sous pression réduite (20 mm de mercure ; 2,7 kPa) à 60 °C. Le produit obtenu (20 g) est chromatographié sur 30 g de gel de silice neutre contenus dans une colonne de 3,8 cm de diamètre.

On élue successivement avec 1 200 $cm^3$ d'un mélange de cyclohexane et d'acétate d'éthyle dont la teneur en acétate d'éthyle varie progressivement de 0 à 10 %, avec 2 400 $cm^3$ d'un mélange cyclohexane-acétate d'éthyle (90-10 en volumes), avec 1 080 $cm^3$ d'un mélange cyclohexane-acétate d'éthyle (85-15 en volumes), avec 1 080 $cm^3$ d'un mélange cyclohexane-acétate d'éthyle (85-15 en volumes) avec 1080 $cm^3$ d'un mélange cyclohexane-acétate d'éthyle (80-20 en volumes) et avec 1 440 $cm^3$ d'un mélange cyclohexane-acétate d'éthyle (70-30 en volumes), en recueillant des fractions de 120 $cm^3$. Les fractions 51, 52 et 53 sont réunies et concentrées à sec sous pression réduite (20 mm de mercure ; 2,7 kPa) à 55 °C. Le produit obtenu (1,3 g) est dissous dans 17 $cm^3$ d'éthanol bouillant ; la solution est additionnée de 0,4 g de noir décolorant puis filtrée à chaud et conservée, après refroidissement, pendant 3 heures à une température voisine de 5 °C. Les cristaux apparus sont séparés par filtration, lavés par 1 $cm^3$ d'éthanol et 2 fois par 2 $cm^3$ au total d'oxyde d'isopropyle. Après séchage sous pression réduite (1 mm de mercure ; 0,13 kPa) à 40 °C, on obtient 0,8 g de N-méthyl (quinolyl-3)-2 tétrahydrothiophènecarbothioamide-2 fondant à 159 °C.

Le sulfure de quinolyl-3 méthyle et de chloro-3 propyle peut être préparé de la façon suivante :

A une solution refroidie à 1 °C de 59,2 g de dichlorhydrate de (quinolyl-3 méthyl)-2 isothiourée dans 100 $cm^3$ d'eau distillée on ajoute, en 9 minutes et en laissant la température remonter à 10 °C, 40,8 $cm^3$ d'une solution aqueuse de soude 10 N. Après chauffage pendant 20 minutes à une température voisine de 70 °C puis refroidissement à 12 °C, on ajoute, goutte à goutte en 5 minutes, 25 $cm^3$ d'une solution aqueuse de soude 10 N. On ajoute ensuite 33,6 g de bromo-1 chloro-3 propane et poursuit l'agitation pendant 20 heures à une température voisine de 20 °C. Le mélange réactionnel est alors extrait 3 fois par 400 $cm^3$ au total de chlorure de méthylène. Les extraits organiques sont réunis, lavés par 100 $cm^3$ d'eau distillée, séchés sur du sulfate de sodium anhydre et concentrés sous pression réduite (20 mm de mercure ; 2,7 kPa) à 35 °C de façon à ramener le volume à 100 $cm^3$ environ. La solution ainsi obtenue est chromatographiée sur 250 g de gel de silice neutre contenus dans une colonne de 3,8 cm de diamètre. On élue avec 1 500 $cm^3$ de chlorure de méthylène en recueillant 1 fraction de 600 $cm^3$ et 1 fraction de 900 $cm^3$. Cette dernière est concentrée à sec sous pression réduite (20 mm de mercure ; 2,7 kPa) à 35 °C. On obtient ainsi 9,2 g de sulfure de quinolyl-3 méthyle et de chloro-3 propyle sous la forme d'une huile jaune.

(Rf = 0,6 ; chromatographie sur couche mince de gel de silice ; solvant : acétate d'éthyle).

Le dichlorhydrate de (quinolyl-3 méthyl)-2 isothiourée peut être préparé de la façon suivante :

A 19,7 g de thiourée en suspension dans 110 $cm^3$ d'éthanol bouillant, on ajoute, goutte à goutte en 10 minutes, 46,4 g de chlorhydrate de chlorométhyl-3 quinoléine en solution dans 160 $cm^3$ d'éthanol à 70 °C. Le mélange réactionnel est agité pendant 1 heure 30 minutes à l'ébullition puis refroidi à 10 °C. Les cristaux qui se sont formés sont séparés par filtration, lavés 2 fois par 100 $cm^3$ au total d'éthanol et séchés sous pression réduite (20 mm de mercure ; 2,7 kPa) à une température voisine de 20 °C. On obtient ainsi 59,3 g de dichlorhodrate de (quinolyl-3 méthyl)-2 isothiourée fondant à 226-228 °C.

Le chlorhydrate de chlorométhyl-3 quinoléine peut être préparé selon la méthode décrite par J. KOTLER-BRAJTBURG, Acta Pol. Pharm. *25* (4), 383 (1968) ; CA *70*, 87518 s.

La présente invention concerne également les médicaments constitués par au moins un produit de formule générale (I), à l'état pur ou sous forme d'une composition dans laquelle il est associé à tout autre produit pharmaceutiquement compatible, pouvant être inerte ou physiologiquement actif. Les médicaments selon l'invention peuvent être utilisés par voie orale, parentérale ou rectale.

Comme compositions solides pour administration orale peuvent être utilisés des comprimés, pilules, poudres (notamment dans des capsules de gélatine ou des cachets) ou granulés. Dans ces compositions, le produit actif selon l'invention est mélangé à un ou plusieurs diluants inertes, tels que amidon, cellulose, saccharose, lactose ou silice. Ces compositions peuvent également comprendre des substances autres que les diluants, par exemple un ou plusieurs lubrifiants tel que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent également comprendre des substances autres que les diluants, par exemple des produits mouillants, édulcorants, épaississants, aromatisants ou stabilisants.

Les compositions stériles pour administration parentérale peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont les suppositoires ou les capsules rectales, qui contiennent outre le produit actif des excipients tels que le beurre de cacao, des glycérides semi-synthétiques ou des polyéthylèneglycols.

En thérapeutique humaine, les produits de l'invention sont, selon la définition du symbole Het donnée précédemment, particulièrement utiles dans le traitement des ulcères gastrointestinaux et, dans le traitement de l'hypertension. Les doses dépendent de l'effet recherché et de la durée du traitement ; elles sont généralement comprises entre 25 et 1 000 mg par jour par voie orale pour un adulte en une ou plusieurs prises.

D'une façon générale, le médecin déterminera la posologie qu'il estime la plus appropriée en fonction de l'âge, du poids et de tous les autres facteurs propres au sujet à traiter.

L'exemple suivant, donné à titre non limitatif, illustre des compositions selon l'invention.

Exemple

On prépare selon la technique habituelle des comprimés dosés à 25 mg de produit actif ayant la composition suivante :

| | |
|---|---|
| N-méthyl(pyridyl-3)-2 tétrahydrothiopyrannecarbothioamide-2 | 25 mg |
| amidon | 60 mg |
| silice colloïdale | 50 mg |
| stéarate de magnésium | 2 mg |

**Revendications** (pour les Etats contractants : BE, CH, DE, FR, GB, IT, LI, LU, NL, SE)

1. Un dérivé de la thioformamide caractérisé en ce qu'il répond à la formule générale :

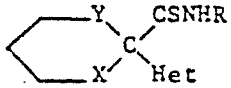

dans laquelle R représente un atome d'hydrogène ou un radical alcoyle contenant 1 à 4 atomes de carbone en chaîne droite ou ramifiée, Het représente un radical hétérocyclique à caractère aromatique contenant un ou deux atomes d'azote choisi parmi pyridyle-3, pyridyle-4, pyridazinyle-3, pyrazinyle, ou quinolyle-3, X représente un atome de soufre ou d'oxygène et Y représente un atome de soufre ou d'oxygène, une liaison de valence ou un radical méthylène.

2. Un dérivé de la thioformamide selon la revendication 1, caractérisé en ce que le radical Het représente pyridyle-3 ou quinolyle-3.

3. Procédé de préparation d'un produit selon la revendication 1 caractérisé en ce que l'on fait réagir une amine de formule générale :

$$R—NH_2$$

dans laquelle R est défini comme dans la revendication 1 sur un dithioester de formule générale :

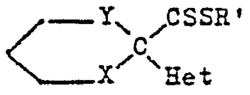

dans laquelle les symboles Het, X et Y sont définis comme dans la revendication 1 et R' représente un radical alcoyle contenant 1 à 4 atomes de carbone en chaîne droite ou ramifiée ou un radical benzyle ou carboxyméthyle, puis isole le produit obtenu.

4. Procédé de préparation d'un produit selon la revendication 1 dans la formule duquel R est un radical alcoyle contenant 1 à 4 atomes de carbone en chaîne droite ou ramifiée caractérisé en ce que, sur un dérivé de formule générale :

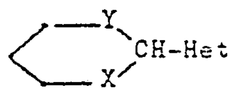

dans laquelle X, Y et Het sont définis comme dans la revendication 1, on fait réagir un dérivé organo-lithien puis un isothiocyanate de formule générale :

R'''—N=C=S

dans laquelle R''' représente un radical alcoyle contenant 1 à 4 atomes de carbone en chaîne droite ou ramifiée, puis isole le produit obtenu.

5. Médicament caractérisé en ce qu'il consiste en un produit selon la revendication 1.

6. Composition pharmaceutique caractérisée en ce qu'elle contient à titre de produit actif au moins un produit selon la revendication 1 éventuellement en association avec un ou plusieurs diluants ou adjuvants compatibles et pharmaceutiquement acceptables.

**Revendication** (pour l'Etat contractant AT)

Procédé de préparation d'un dérivé de la thioformamide de formule générale :

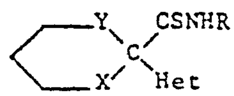

dans laquelle R représente un atome d'hydrogène ou un radical alcoyle contenant 1 à 4 atomes de carbone en chaîne droite ou ramifiée, Het représente un radical hétérocyclique à caractère aromatique contenant un ou deux atomes d'azote choisi parmi pyridyle-3, pyridyle-4, pyridazinyle-3, pyrazinyle ou quinolyle-3, X représente un atome de soufre ou d'oxygène et Y représente un atome de soufre ou d'oxygène, une liaison de valence ou un radical méthylène, caractérisé en ce que l'on fait réagir une amine de formule générale :

$R—NH_2$

dans laquelle R a la définition correspondante sur un dithioester de formule générale :

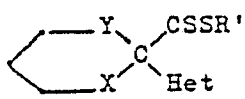

dans laquelle les symboles Het, X et Y sont définis comme précédemment et R' représente un radical alcoyle contenant 1 à 4 atomes de carbone en chaîne droite ou ramifiée ou un radical benzyle ou carboxyméthyle, puis isole le produit obtenu,
ou, lorsque R est un radical alcoyle contenant 1 à 4 atomes de carbone en chaîne droite ou ramifiée en ce que, sur un dérivé de formule générale :

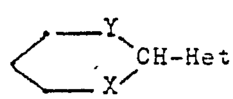

dans laquelle X, Y et Het sont définis comme précédemment, on fait réagir un dérivé organo-lithien puis un isothiocyanate de formule générale :

R'''—N=C=S

dans laquelle R''' représente un radical alcoyle contenant 1 à 4 atomes de carbone en chaîne droite ou ramifiée, puis isole le produit obtenu.

**Claims** (for the Contracting States : BE, CH, DE, FR, GB, IT, LI, LU, NL, SE)

1. A derivative of thioformamide characterised in that it corresponds to the general formula :

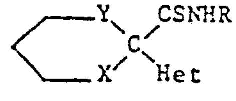

in which R denotes a hydrogen atom or an alkyl radial containing 1 to 4 carbon atoms as a straight or branched chain, Het denotes a heterocyclic radical of aromatic nature containing one or two nitrogen atoms chosen from 3-pyridyl, 4-pyridyl, 3-pyridazinyl, pyrazinyl, or 3-quinolyl, X denotes a sulphur or oxygen atom and Y denotes a sulphur or oxygen atom, a valency bond or a methylene radical.

2. A derivative of thioformamide according to Claim 1, characterised in that the radical Het denotes 3-pyridyl or 3-quinolyl.

3. Process for preparing a product according to Claim 1, characterised in that an amine of general formula :

$$R—NH_2$$

in which R is defined as in Claim 1 is reacted with a dithioester of general formula :

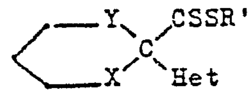

in which the symbols Het, X and Y are defined as in Claim 1, and R′ denotes an alkyl radical containing 1 to 4 carbon atoms as a straight or branched chain or a benzyl or carboxymethyl radical, and the product obtained is then isolated.

4. Process for preparing a product according to Claim 1, in the formula of which R is an alkyl radical containing 1 to 4 carbon atoms as a straight or branched chain, characterised in that a derivative of general formula :

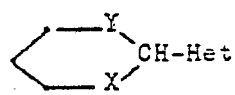

in which X, Y and Het are defined as in Claim 1 is reacted with an organolithium derivative and then with an isothiocyanate of general formula :

$$R'''—N{=}C{=}S$$

in which R‴ denotes an alkyl radical containing 1 to 4 carbon atoms as a straight or branched chain, and the product obtained is then isolated.

5. Medicament characterised in that it consists of a product according to Claim 1.

6. Pharmaceutical composition characterised in that it contains as active product at least one product according to Claim 1, if appropriate in combination with one or more compatible and pharmaceutically acceptable diluents or adjuvants.

**Claim** (for the Contracting State AT)

Process for preparing a derivative of thioformamide of general formula :

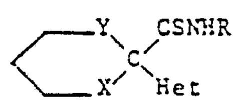

in which R denotes a hydrogen atom or an alkyl radical containing 1 to 4 carbon atoms as a straight or branched chain, Het denotes a heterocyclic radical of aromatic nature containing 1 or 2 nitrogen atoms, chosen from 3-pyridyl, 4-pyridyl, 3-pyridazinyl, pyrazinyl or 3-quinolyl, X denotes a sulphur or oxygen atom and Y denotes a sulphur or oxygen atom, a valency bond or a methylene radical, characterised in that an amine of general formula :

$$R—NH_2$$

in which R has the corresponding definition is reacted with a dithioester of general formula :

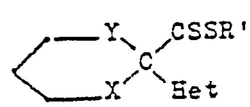

in which the symbols Het, X and Y are defined as previously and R′ denotes an alkyl radical containing 1 to 4 carbon atoms as a straight or branched chain or a benzyl or carboxymethyl radical, and the product obtained is then isolated,

or, when R is an alkyl radical containing 1 to 4 carbon atoms as a straight or branched chain, in that a derivative of general formula :

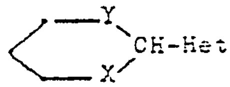

in which X, Y and Het are defined as previously is reacted with an organolithium derivative and then an isothiocyanate of general formula :

R'''—N=C=S

in which R''' denotes an alkyl radical containing 1 to 4 carbon atoms as a straight or branched chain, and the product obtained is then isolated.

**Patentansprüche** (für die Vertragsstaaten : BE, CH, DE, FR, GB, IT, LI, LU, NL, SE)

1. Ein Derivat des Thioformamids, dadurch gekennzeichnet, daß es der allgemeinen Formel

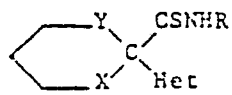

entspricht, worin R ein Wasserstoffatom oder einen Alkylrest mit 1 bis 4 Kohlenstoffatomen in gerader oder verzweigter Kette bedeutet, Het einen heterocyclischen Rest mit aromatischem Charakter enthaltend 1 oder 2 Stickstoffatome ausgewählt unter Pyridyl-3, Pyridyl-4, Pyridazinyl-3, Pyrazinyl oder Chinolyl-3, bedeutet, X ein Schwefel- oder Sauerstoffatom ist und Y ein Schwefel- oder Sauerstoffatom, eine Valenzbindung oder einen Methylenrest bedeutet.

2. Ein Derivat des Thioformamids gemäß Anspruch 1, dadurch gekennzeichnet, daß der Rest Het Pyridyl-3 oder Chinolyl-3 bedeutet.

3. Verfahren zur Herstellung eines Produkts gemäß Anspruch 1, dadurch gekennzeichnet, daß man ein Amin der allgemeinen Formel

$$R—NH_2$$

worin R wie in Anspruch 1 definiert ist, auf einen Dithioester der allgemeinen Formel

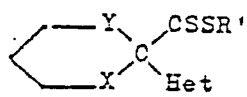

worin die Symbole Het, X und Y wie in Anspruch 1 definiert sind und R' einen Alkylrest mit 1 bis 4 Kohlenstoffatomen in gerader oder verzweigter Kette oder einen Benzyl- oder Carboxymethylrest bedeutet, einwirken läßt und dann das erhaltene Produkt isoliert.

4. Verfahren zur Herstellung eines Produkts gemäß Anspruch 1, in dessen Formel R ein Alkylrest mit 1 bis 4 Kohlenstoffatomen in gerader oder verzweigter Kette ist, dadurch gekennzeichnet, daß man auf ein Derivat der allgemeinen Formel

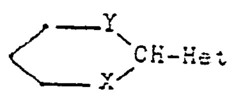

worin X, Y und Het wie in Anspruch 1 definiert sind, ein Organolithium-Derivat, und dann ein Isothiocyanat der allgemeinen Formel

R'''—N=C=S

worin R''' einen Alkylrest mit 1 bis 4 Kohlenstoffatomen in gerader oder verzweigter Kette bedeutet, einwirken läßt und dann das erhaltene Produkt isoliert.

5. Arzneimittel, dadurch gekennzeichnet, daß es aus einem Produkt gemäß Anspruch 1 besteht.

6. Pharmazeutische Zusammensetzung, dadurch gekennzeichnet, daß sie als aktives Produkt mindestens ein Produkt gemäß Anspruch 1, gegebenenfalls zusammen mit einem oder mehreren verträglichen und pharmazeutisch annehmbaren Verdünnungsmitteln oder Hilfsmitteln enthält.

**Patentanspruch** (für den Vertragsstaat AT)

Verfahren zur Herstellung eines Thioformamidderivats der allgemeinen Formel :

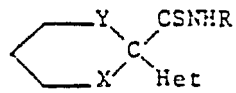

in welcher R ein Wasserstoffatom oder einen Alkylrest mit 1 bis 4 Kohlenstoffatomen in gerader oder verzweigter Kette darstellt, Het einen aromatischen heterocyclischen Rest mit einem oder zwei Stickstoffatomen, ausgewählt aus 3-Pyridyl, 4-Pyridyl, 3-Pyridazinyl, Pyrazinyl oder 3-Chinolyl, darstellt, X ein Schwefel- oder Sauerstoffatom darstellt und Y ein Schwefel- oder Sauerstoffatom, eine Valenzbindung oder einen Methylenrest darstellt, dadurch gekennzeichnet, daß man ein Amin der allgemeinen Formel :

$$R-NH_2$$

in welcher R die entsprechende Bedeutung hat, mit einem Dithioester der allgemeinen Formel :

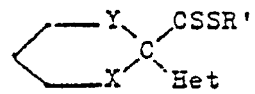

in welcher die Symbole Het, X und Y die obige Bedeutung haben und R' einen Alkylrest mit 1 bis 4 Kohlenstoffatomen in gerader oder verzweigter Kette oder einen Benzyl- oder Carboxymethylrest darstellt, reagieren läßt und dann die erhaltene Verbindung isoliert, oder, wenn R ein Alkylrest mit 1 bis 4 Kohlenstoffatomen in gerader oder verzweigter Kette ist, daß man auf eine Verbindung der allgemeinen Formel :

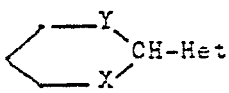

in welcher X, Y und Het die obige Bedeutung haben, ein Organolithiumderivat und dann ein Isothiocyanat der allgemeinen Formel :

$$R'''-N=C=S$$

in welcher R''' einen Alkylrest mit 1 bis 4 Kohlenstoffatomen in gerader oder verzweigter Kette darstellt, einwirken läßt und dann die erhaltene Verbindung isoliert.